(19) Europäisches Patentamt
European Patent Office
Office européen des brevets

(11) Veröffentlichungsnummer : **0 134 200**
**B1**

# (12) EUROPÄISCHE PATENTSCHRIFT

(45) Veröffentlichungstag der Patentschrift : **04.03.87**

(51) Int. Cl.[4] : **C 07 C148/00**

(21) Anmeldenummer : **84810386.7**

(22) Anmeldetag : **06.08.84**

## (54) Verfahren zur Herstellung von Merkaptanen.

(30) Priorität : **12.08.83 CH 4420/83**

(43) Veröffentlichungstag der Anmeldung : **13.03.85 Patentblatt 85/11**

(45) Bekanntmachung des Hinweises auf die Patenterteilung : **04.03.87 Patentblatt 87/10**

(84) Benannte Vertragsstaaten : **CH DE FR GB IT LI NL**

(56) Entgegenhaltungen :
**FR-A- 2 008 330**
**HOUBEN-WEYL: "Methoden der organischen Chemie", 4. Auflage, Band IV/1c, Reduktion, Teil 1, Seiten 486-487, Georg Thieme Verlag, Stuttgart, DE;**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Bader, Rolf**
**Mühlestiegrain 7**
**CH-4125 Riehen (CH)**
Erfinder : **Blaser, Hans-Ulrich, Dr.**
**Brücklismattstrasse 18**
**CH-4107 Ettingen (CH)**
Erfinder : **Thies, Hans, Dr.**
**Tulpenweg 9**
**D-7888 Rheinfelden-Degerfelden (DE)**

**EP 0 134 200 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von aliphatischen Merkaptanen durch katalytische Hydrierung von festen aliphatischen Disulfiden in der Schmelzphase.

Bei der Herstellung und destillativen Trennung von Merkaptanen bilden sich Disulfide als unerwünschte Nebenprodukte. Während niedermolekulare flüssige und lösliche Disulfide mit klassischen Methoden zu Merkaptanen reduziert werden können, ist für höhermolekulare feste aliphatische Disulfide noch keine befriedigende Reduktionsmethode aufgezeigt worden, besonders wenn es sich um schwerlösliche oder unlösliche Produkte handelt. Die klassischen Reduktionsmethoden z. B. mittels Zink oder Eisen in mineralsaurem Medium, sind ausserdem häufig nicht selektiv genug, da gleichzeitig eine Hydrogenolyse der gebildeten Merkaptane zu Kohlenwasserstoffen stattfindet.

Disulfide und Merkaptane sind bekannterweise (Houben-Weyl, 4/1c (1980), Seite 486, 487) starke Katalysatorgifte. Es sind daher nur einige katalytische Hydrierungen von aliphatischen Disulfiden beschrieben worden. Diese Reaktionen werden unter milden Bedingungen in Lösung durchgeführt. Bei Verwendung von Platinmetallen als Katalysatoren werden niedrige Temperaturen angewendet. Ferner müssen teilweise hohe Mengen Edelmetallkatalysatoren eingesetzt werden, um den Aktivitätsverlust an Katalysator auszugleichen und während zufriedenstellender Reaktionszeiten gute Ausbeuten zu erzielen. Die Bildung von Dodecylmercaptan durch Hydrierung von Didodecyldisulfid in Gegenwart von Molybdänsulfidkatalysatoren ist in CA 54, 19466 (1960) beschrieben. Zur Erzielung hoher Ausbeuten werden hohe Temperaturen und hohe Drücke benötigt, was als Nachteil empfunden wird.

Die FR-A-2 008 330 beschreibt ein Verfahren zur Herstellung von halogenierten Phenylmerkaptanen, bei dem halogenierte Diphenyldisulfide in Gegenwart geringer Mengen eines Edelmetallkatalysators unter Druck mit Wasserstoff in der Reaktionsschmelze hydriert werden.

Gegenstand der Erfindung ist ein Verfahren zur Herstellung von aliphatischen Merkaptanen durch katalytische Hydrierung von festen aliphatischen Disulfiden in Gegenwart von 0,01 bis 3 Gew.-%, bezogen auf das Disulfid, eines Metallkatalysators aus der Platinmetallgruppe, das dadurch gekennzeichnet ist, dass die Hydrierung in der Schmelze der Disulfide durchgeführt wird.

Feste aliphatische Disulfide sind dem Fachmann bekannt. Es kann sich z. B. um cycloaliphatische Disulfide und aliphatische Disulfide wie z. B. Dibenzyldisulfid und Dialkyldisulfide handeln. Solche Disulfide werden bei der Mercaptanherstellung oft als Nebenprodukte gebildet. Unter den aliphatischen Disulfiden sind solche bevorzugt, deren α-C-Atome an der Disulfidbrücke unverzweigt sind.

Eine bevorzugte Gruppe von festen Disulfiden sind Di-(perfluoralkyl-alkyl) disulfide, die z. B. bei der Herstellung von Lodyne®-$R_f$-Mercaptanen als Nebenprodukte entstehen. Diese Disulfide entsprechen bevorzugt der Formel

$$F(CF_2)_a—R^1—S—S—R^2—(CF_2)_b\ F \qquad (I)$$

worin a und b unabhängig voneinander eine Zahl von 1-50 bedeuten und $R^1$ und $R^2$ unabhängig voneinander Alkylen sind, das durch O, S oder $—R^3N—$ unterbrochen sein kann, worin $R^3$ für H oder Alkyl mit 1-12 C-Atomen steht, wobei das Disulfid insgesamt mindestens 10 C-Atome aufweist.

Bei den Zahlen a und b handelt es sich bevorzugt um gerade Zahlen. Der Zahlenwert von a und b beträgt bevorzugt 1 bis 30, besonders 4 bis 20. Die $(CF_2)_a$- bzw. $(CF_2)_b$-Ketten können ferner durch O-Atome unterbrochen sein. Solche Perfluoralkyloxy-perfluoralkylgruppen enthalten bevorzugt 4 bis 20 C-Atome.

Bei $R^1$ und $R^2$ kann es sich um lineares Alkylen mit 1 bis 12, besonders 2 bis 6 und insbesondere 2 bis 4 C-Atomen handeln. Besonders sind $R^1$ und $R^2$ Aethylen. $R^1$ und $R^2$ können auch lineares oder verzweigtes Alkylenthioalkylen, Alkylenoxaalkylen und Alkyleniminoalkylen mit bevorzugt 2 bis 12 C-Atomen sein. $R^3$ im Alkyleniminoalkylen kann ein Wasserstoffatom oder Alkyl mit bevorzugt 1 bis 6 C-Atomen bedeuten.

Es hat sich als zweckmässig erwiesen, die Hydrierung oberhalb der Schmelztemperatur der Disulfide durchzuführen, z. B. mindestens 20 °C über deren Schmelztemperatur. Vorteilhaft beträgt die Temperatur 100 bis 200 °C, besonders 120 bis 180 °C.

Die Hydrierung kann auch in einer Dispersion der Schmelze in einem geeigneten Dispergiermittel durchgeführt werden. Geeignete Dispergiermittel sind z. B. organische Lösungsmittel, die keine oder nur geringe Anteile der aliphatischen Disulfide zu lösen vermögen. Ein bevorzugtes Dispergiermittel ist Wasser. Das Dispergiermittel kann z. B. in einer Menge bis zu 90, vorzugsweise bis zu 50 Gew.-% zugegeben werden, bezogen auf die aliphatischen Disulfide.

Die Hydrierung wird vorteilhaft unter Ueberdruck von vorzugsweise mindestens 5 bar durchgeführt. Es ist ein besonderer Vorteil des erfindungsgemässen Verfahrens, dass die Reaktion in wässriger Dispersion schon bei relativ niedrigem Druck in kurzen Zeiten praktisch quantitativ abläuft. Bevorzugt wird in einem Druckbereich von 5 bis 80 bar hydriert, in wässriger Dispersion insbesondere bei 5 bis 20 bar, und in der Schmelze besonders bei 20 bis 80 bar.

Als Katalysatoren sind die Platinmetalle Ru, Rh, Pd, Os, Ir und Pt geeignet. Bevorzugt sind Ru, Pt und besonders Pd. Im allgemeinen werden Katalysatoren eingesetzt, bei denen die Platinmetalle auf festen

Trägermaterialien wie Kieselgel, Aluminiumoxid und besonders Kohlenstoff aufgebracht sind. Die Katalysatoren können mit Schwefelverbindungen wie $H_2S$ partiell inaktiviert sein (sulfidierte Katalysatoren). Beim erfindungsgemässen Verfahren ist der Aktivitäts-verlust nur gering, wenn nicht andere Katalysatorgifte als Merkaptane oder Disulfide zugegen sind und der eingesetzte Katalysator kann überraschend wieder verwendet werden. Vorteilhaft wird ein Teil des gebrauchten durch frischen Katalysator ersetzt oder ergänzt, z. B. 5 bis 50, besonders 10 bis 30 Gew.-%, bezogen auf den gebrauchten Katalysator. Der Katalysator selbst wird bevorzugt in einer Menge von 0,05 bis 3, besonders 0,1 bis 3 Gew.-% Platinmetalle eingesetzt, bezogen auf das Disulfid.

Das erfindungsgemässe Verfahren wird in hierfür üblichen Hydrierapparaten wie z. B. Autoklaven durchgeführt, die vorteilhaft über Einrichtungen zum ständigen guten Durchmischen der Reaktionspartner verfügen. Im einzelnen kann man so vorgehen, dass man das aliphatische Disulfid, den Katalysator und gegebenenfalls ein Dispergiermittel vorlegt, die Luft verdrängt und danach Wasserstoff aufpresst. Dann heizt man das Reaktionsgemisch bis zum Schmelzen auf bzw. stellt die gewünschte Reaktionstemperatur ein und hydriert, bis keine Wasserstoffaufnahme mehr erfolgt.

Zur Isolierung der gebildeten Merkptane kann der Katalysator von der Reaktionsschmelze z. B. durch eine Druckfiltration in einem beheizten Filter entfernt werden. Es kann aber auch das Reaktionsgemisch mit einem geeigneten Lösungsmittel aufgenommen werden, der Katalysator abfiltriert und danach das Lösungsmittel wieder entfernt werden. Gewünschtenfalls können die Merkaptane anschliessend weiter gereinigt werden, z. B. mit destillativen Methoden oder durch Umkristallisation.

Mit dem erfindungsgemässen Verfahren werden Mercaptane auf wirtschaftliche und umweltfreundliche Weise erhalten. Die Reaktionszeiten sind kurz. Die überraschend hohe Selektivität der Hydrierung — es wird weder eine $H_2S$-Abspaltung noch im Falle von Perfluoralkyldisulfiden eine HF-Abspaltung beobachtet — führt zu fast quantitativen Ausbeuten (über 95 %) der gewünschten Mercaptane. Ueberraschend können relativ geringe Katalysatorenmengen eingesetzt werden, und es wird auch nur eine geringe Katalysatordesaktivierung beobachtet, was sogar die Wiederverwedung des gebrauchten Katalysators erlaubt. Ferner ermöglicht es das Verfahren, bei der Mercaptanherstellung anfallende Disulfidrückstände wieder in die gewünschten Mercaptane zu überführen.

Obwohl solche Rückstände oft mit Katalysatorgiften wie z. B. Schwermetallsalzen angereichert sind, werden auch hier mit der Hydrierung hohe Ausbeuten in kurzen Reaktionszeiten mit oft nur geringen Katalysatordesaktivierung erzielt.

Die Mercaptane können zur Herstellung von Detergentien verwendet werden. Fluorierte Mercaptane dienen z. B. als Löschmittel für Oelbrände auf Wasseroberflächen, als Zusatz zu Papiertellern oder zur Textilveredelung.

Die nachfolgenden Beispiele erläutern die Erfindung.

### Beispiel 1

In einem 60 l-Autoklav werden 30 kg Lodyne®-$R_f$-Disulfide (eine Mischung von Perfluoralkyläthyldisulfiden der Kettenlänge $C_8$-$C_{40}$) mit 10 kg Wasser und 0,75 kg Palladium-Kohle-Katalysator (0,038 kg Palladium) vorgelegt. Nach dem Verdrängen der Luft durch mehrmaliges Aufpressen und Entlasten von jeweils 5 bar $N_2$ und dem Verdrängen des Stickstoffs auf analoge Weise mit Wasserstoff wird der Wasserstoffdruck über ein Druckreduzierventil auf 7,5 bar eingestellt. Das Gemisch wird aufgeheizt, zum Schmelzen gebracht und der Rührer mit einer Drehzahl von 500 $min^{-1}$ in Gang gesetzt. Bei einer Reaktionstemperatur von 140-150 °C und einem Gesamtdruck von ca. 10 bar ist die Hydrierung nach 5 Stunden beendet. Der Rührer wird abgestellt, die Temperatur wird 6,5 Stunden auf 140 °C gehalten, damit sich der Katalysator in der wässrigen Phase absetzen kann. Das Reaktionsgemisch wird auf 120 °C abgekühlt und der $H_2$-Druck über eine NaOH-Absorptionsanlage ins Freie entlastet. Durch 5-maliges Aufpressen und Entlasten von jeweils 5 bar $N_2$ wird der Wasserstoff aus dem Autoklaven gespült. Ueber ein dampfbeheiztes Steigrohr und Druckfilter wird die Schmelze abfiltriert und in einer Vorlage zum Erstarren gebracht. Die Reinheit der erhaltenen Perfluoralkyl-äthylmercaptane beträgt etwa 95 %. Die Hydrogenolyse von SH-Gruppen liegt unterhalb 0,2 %, die Hydrogenolyse von Fluor unterhalb 0,1 %.

### Beispiel 2

Verfährt man wie in Beispiel 1, aber ohne Zusatz von Wasser, dann erhält man ebenfalls Perfluoralkyläthylmercaptane mit einer Reinheit von 95 %.

### Beispiel 3

Man verfährt wie in Beispiel 1, aber unter Zusatz von Toluol oder Xylol anstelle von Wasser. Das Verhältnis von Disulfid zum Dispergiermittel beträgt je 1 : 1. Die Filtration erfolgt bei etwa 80 °C. Die Ausbeute an Perfluoralkyläthylmercaptanen liegt über 90 %. Eine Kernhydrierung der verwendeten Dispergiermittel wird nicht beobachtet.

### Beispiel 4

Die Reaktion gemäss Beispiel 2 wird bei 50 bar wiederholt. Die Anwendung höherer Drücke bei verunreinigten schwer hydrierbaren Perfluoralkyläthyldisulfiden ist hierbei besonders vorteilhaft. Bei einem Druck von 50 bar verläuft die Hydrierung innerhalb von 1/2 Stunde während des Aufheizens der Schmelze auf 150 °C. Die Reinheit der Merkaptane beträgt 95 %.

Beispiele 5 und 6

Die Reaktion gemäss Beispiel 4 wird wiederholt, wobei die Katalysatormenge auf die Hälfte (1,25 Gew.-%) herabgesetzt wird.
Druck 50 bar, Hydrierzeit ca. 3 h, Reinheit Merkaptane 94 %
Druck 80 bar, Hydrierzeit ca. 2 h, Reinheit Merkaptane 93 %

Beispiele 7, 8, 9

Verfährt man wie in Beispiel 1 unter Verwendung folgender Edelmetall-Katalysatoren, so erhält man :
2,5 Gew.% Pt/C 5 %, Hydrierzeit 8h, Reinheit Merkaptane 92 %
2,5 Gew.% Pt/C 5 % sulfidiert, Hydrierzeit 3h, Reinheit Merkaptane 92 %
5 Gew.% Ru/C 5 %, Hydrierzeit 10h, Reinheit Merkaptane 89 %

Beispiel 10

113 g Dibenzyldisulfid (Reinheit 98 %, Schmelzpkt. 68-70°) werden zusammen mit 50 g Wasser und 5,5 g Pd/C 5 % vorgelegt. Die Hydrierung verläuft mit hoher Selektivität bei 120 °C und 20 bar innerhalb 22 h. Die Ausbeute an Benzylmerkaptan beträgt 22 % der Theorie neben 75 % noch nicht hydriertem Dibenzyldisulfid für das nicht optimierte Verfahren.

Beispiel 11

45 g Dioctadecyldisulfid (Reinheit 90 %, Schmelzpunkt 60-62 °C) werden mit 150 g Wasser und 2,25 g Pd/C 5 % in einem Autoklaven vei 150 °C und 20 bar innert 1 h hydriert. Die Ausbeute an Octadecylmerkaptan beträgt 96 % der Theorie.

Beispiel 12

82,5 g Didecyldisulfid (Reinheit 97 %) werden mit einer Mischung von 15 g tert. Didodecyldisulfiden (Disulfidbrücke sowohl am endständigen als auch am mittelständigen tert.C-Atom) und 4,8 g Pd/C 5 % bei 100-200 °C und 22 bar während 15 Std. hydriert. Während das Didecyldisulfid zu mehr als 95 % zum Decylmerkaptan hydriert wird, verbleiben die tert.-Dodecyldisulfide praktisch unhydriert zurück.

**Patentansprüche**

1. Verfahren zur Herstellung von aliphatischen Merkaptanen durch katalytische Hydrierung von festen aliphatischen Disulfiden in Gegenwart von 0,01 bis 3 Gew.-%, bezogen auf das Disulfid, eines Metallkatalysators aus der Platinmetallgruppe, dadurch gekennzeichnet, dass die Hydrierung in der Schmelze der Disulfide durchgeführt wird.
2. Verfahren gemäss Anspruch 1, dadurch gekennzeichnet, dass die Hydrierung oberhalb der Schmelztemperatur der Disulfide durchgeführt wird.
3. Verfahren gemäss Anspruch 2, dadurch gekennzeichnet, dass die Temperatur der Schmelze mindestens 20 °C über der Schmelztemperatur liegt.
4. Verfahren gemäss Anspruch 3, dadurch gekennzeichnet, dass die Reaktionstemperatur 100 bis 200 °C beträgt.
5. Verfahren gemäss Anspruch 1, dadurch gekennzeichnet, dass die Hydrierung in der Schmelze in Gegenwart von Wasser oder einem inerten organischen Lösungsmittel als Dispersionsmittel durchgeführt wird.
6. Verfahren gemäss Anspruch 1, dadurch gekennzeichnet, dass die Hydrierung bei einem Druck von mindestens 5 bar durchgeführt wird.
7. Verfahren gemäss Anspruch 1, dadurch gekennzeichnet, dass die Hydrierung in Gegenwart eines sulfidierten oder nichtsulfidierten Ruthenium-, Platin- oder besonders Palladiumkatalysators, der auf ein Trägermaterial aufgebracht ist, durchgeführt wird.
8. Verfahren gemäss Anspruch 1, dadurch gekennzeichnet, dass der Katalysator unter Ersatz eines Teiles davon durch frischen Katalysator wiederverwendet wird bzw. durch einen Teil frischen Katalysator ergänzt wird.
9. Verfahren gemäss Anspruch 1, dadurch gekennzeichnet, dass das feste Disulfid der Formel

$$F(CF_2)_a—R^1—S—S—R^2—(CF_2)_bF \quad (I)$$

entspricht, worin a und b unabhängig voneinander eine Zahl von 1 bis 50 bedeuten und $R^1$ und $R^2$ unabhängig voneinander Alkylen sind, das durch O,S oder $—R^3N—$ unterbrochen sein kann, worin $R^3$ für H oder Alkyl mit 1 bis 12 C-Atomen steht, wobei das Disulfid insgesamt mindestens 10 C-Atome aufweist.

10. Verfahren gemäss Anspruch 9, dadurch gekennzeichnet, dass $R^1$ und $R^2$ für Aethylen stehen.

## Claims

1. A process for the preparation of an aliphatic mercaptan by catalytic hydrogenation of a solid aliphatic disulfide in the presence of 0.01 to 3 % by weight, based on said disulfide, of a metal catalyst selected from the group of the platinum metals, which process comprises carrying out the hydrogenation in the melt of the disulfide.

2. A process according to claim 1, wherein the hydrogenation is carried out above the melting point of the disulfude.

3. A process according to claim 2, wherein the temperature of the melt is at least 20 °C above the melting point.

4. A process according to claim 3, wherein the reaction temperature is in the range from 100 to 200 °C.

5. A process according to claim 1, wherein the hydrogenation is carried out in the melt in the presence of water or an inert organic solvent as dispersant.

6. A process according to claim 1, wherein the hydrogenation is carried out under a pressure of at least 5 bar.

7. A process according to claim 1, wherein the hydrogenation is carried out in the presence of a sulfide or non-sulfide ruthenium, platinum or, preferably palladium catalyst which has been applied to a carrier material.

8. A process according to claim 1, wherein the catalyst is reused by replacing a portion thereof with fresh catalyst or adding thereto an amount of fresh catalyst.

9. A process according to claim 1, wherein the solid disulfude has the formula

$$F(CF_2)_a—R^1—S—S—R^2—(CF^2)^bF \quad (I)$$

in which a and b independently of each other are a number from 1 to 50 and $R^1$ and $R^2$ independently of each other are alkylene which can be interrupted by O, S or $—R^3N—$, in which $R^3$ is H or alkyl of 1 to 12 carbon atoms, said disulfide containing a total of not less than 10 carbon atoms.

10. A process according to claim 9, wherein $R^1$ and $R^2$ are ethylene.

## Revendications

1. Procédé de préparation de mercaptans aliphatiques par hydrogénation catalytique de disulfures aliphatiques solides, en présence de 0,01 à 3 % en poids, par rapport au disulfure, d'un catalyseur métallique du groupe des platinoïdes, procédé caractérisé en ce qu'on effectue l'hydrogénation sur la masse fondue des disulfures.

2. Procédé selon la revendication 1 caractérisé en ce qu'on effectue l'hydrogénation à une température supérieure à la température de fusion des disulfures.

3. Procédé selon la revendication 2 caractérisé en ce que la température de la masse fondue est supérieure d'au moins 20 °C à la température de fusion.

4. Procédé selon la revendication 3 caractérisé en ce que la température réactionnelle est comprise entre 100 et 200 °C.

5. Procédé selon la revendication 1 caractérisé en ce que l'hydrogénation est effectuée sur la masse fondue en présence d'eau ou d'un solvant organique inerte jouant le rôle de milieu de dispersion.

6. Procédé selon la revendication 1 caractérisé en ce que l'hydrogénation est effectuée sous une pression d'au moins 5 bar.

7. Procédé selon la revendication 1 caractérisé en ce que l'hydrogénation est effectuée en présence d'un catalyseur au ruthénium, au platine ou, mieux, au palladium, sulfuré ou non, qui est déposé sur une matière support.

8. Procédé selon la revendication 1 caractérisé en ce qu'on réutilise le catalyseur en en remplaçant une partie par du catalyseur frais ou on le complète par une partie de catalyseur frais.

9. Procédé selon la revendication 1 caractérisé en ce que le disulfure solide répond à la formule I :

$$F(CF_2)_a—R^1—S—S—R^2—(CF_2)_bF \quad (I)$$

dans laquelle a et b représentent chacun, indépendamment l'un de l'autre, un nombre de 1 à 50, et $R^1$ et $R^2$ représentent chacun, indépendamment l'un de l'autre, un radical —O— ou —S— alkylène qui peut être interrompu par —O ou —S— ou par un radical —($R^3$)N— dans lequel $R^3$ désigne H ou un alkyle contenant de 1 à 12 atomes de carbone, le disulfure contenant au total au moins 10 atomes de carbone.

10. Procédé selon la revendication 9 caractérisé en ce que $R^1$ et $R^2$ représentent chacun un radical éthylène.